(19) Europäisches Patentamt
European Patent Office
Office européen des brevets

(11) **EP 2 413 129 A1**

(12) **EUROPEAN PATENT APPLICATION**
published in accordance with Art. 153(4) EPC

(43) Date of publication:
**01.02.2012 Bulletin 2012/05**

(51) Int Cl.:
*G01N 21/21* (2006.01)   *G01N 21/45* (2006.01)
*A61B 5/117* (2006.01)

(21) Application number: **09842119.1**

(22) Date of filing: **31.08.2009**

(86) International application number:
**PCT/ES2009/070361**

(87) International publication number:
**WO 2010/109030 (30.09.2010 Gazette 2010/39)**

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **24.03.2009 ES 200930007**

(71) Applicant: **Hanscan IP B.V.
1097JB Amsterdam (NL)**

(72) Inventors:
• **ANTEQUERA DIAZ, Nicolás
E-28108 Alcobendas (Madrid) (ES)**
• **SANCHEZ DEL RIO SAEZ, José
28108 Alcobendas
Madrid (ES)**

(74) Representative: **Temino Ceniceros, Ignacio
Abril Abogados
Amador de los Rios 1-1°
28010 Madrid (ES)**

(54) **LIFE DETECTOR DEVICE**

(57)    Life detector device of the type used for personal identification that uses the evanescent field of the guided light (5) inside a waveguide that preserves the polarization and where this waveguide (8) is configured so that the interaction of the light with the biometric element provokes a change in the polarization of the light as it exits the fibre with respect to the polarization it has when it enters, which is detected by a photo-detector connected to the fibre (10,15); this change in polarization of the guided light is characteristic of the user that is being identified.

**Fig.1**

**Description**

The main objective of this invention is a device that while making use of the evanescent field of the guided light inside a single-mode optical fibre or inside a flat wave guide that can be straight or shaped like an integrated Mach-Zehnder interferometer, preserves the polarization to detect the presence of living human tissue. This device is used as a way to detect that the user that is going to be identified with the biometric system is indeed the correct user and therefore eliminates the possibility of identity theft from occurring.

This invention is considered part of the biometric field, specifically within the evanescent waveguide field of integrated photonics.

BACKGROUND OF THE INVENTION

Currently, there are many fraudulent attempts to steal the identity of a person. Biometrics makes use of the unique biological characteristics of the person for their identification such as the fingerprint, the palm of the hand vein pattern, the cardiac pulse rhythm, etc. In order to conduct the identification, microelectronics, photonics or information technology produce numerous biometric systems each year with the purpose of implementing these as access systems.

Parallel to this increasing number of biometric systems that emerge, a greater number of attempts at falsifying the biometric characteristics are conducted to try to fool the biometric sensor. In this way, by using non-live artificial materials, the hacker attempts to steal the user's identity, who in theory is going to be identified as a registered user of the access system. These attempts to fool the biometric systems causes fear and mistrust in entities that need a high degree of security, like banks, restricted access areas, information or electronic start systems, etc. Therefore, life detection systems that use biometric parameters to prevent the fraudulent use of the systems are necessary.

The PALM VEIN SECURE system from FUJITSU® uses the blood capillary pattern of the palm of the hand as the sole biometric receptor element. An LED set with a near IR emission spectrum illuminates the palm of the hand while a CCD camera captures the image that corresponds to its reflected radiation. This image presents straight and dark curved paths that represent the blood capillary map of the palm and are due to the absorption areas of the near IR radiation emitted by the LEDs that has not been reflected on the palm of the user's hand. The absorption of the near IR radiation by the hand veins corresponds to the deoxygenated haemoglobin in the blood, which represents an absorption peak of 760nm in the electromagnetic spectrum. However, this system has a problem because it is possible to fool it by obtaining a user's palm of the hand characteristic vein pattern and placing this pattern on a characteristic grey coloured paper by using a similar near IR illumination system and a CCD with an absorption band in the near IR.

The biometric sensor with patent number US6813010 captures as a sole biometric element, the veins of a single finger from the user's hand. It detects the near infrared radiation that is transmitted through the finger and emitted by the LED with an emission band in the near IR. The detector optimizes the intensity of the light that reaches the finger and in this way obtains a clearer vein pattern, which helps in its subsequent identification. However, this characteristic vein pattern is easier than the one captured by the FUJISTU palm vein sensor because it captures a smaller area and a capillary network that is less ramified. Because of this, its copying should be easier to perform by using the same falsification procedure as used with the FUJITSU sensor.

To date, only a few life detection systems for biometric sensors have been described in literature. The majority are not implemented in the biometric sensors due to the fact that they can be easily "fooled" by using simple substitutive techniques. So for example, when the human pulse is optically detected it can be easily substituted by using a sheet of paper of a characteristic colour that interrupts the light beam that reaches the detector. Another example can be the measuring of the bio-impedance of the human tissue with an impedance meter. This measurement can be obtained using a material that has impedance that is typical of the characteristic biological element. On the other hand, other life detection systems that are more advanced and measure the roughness of the skin, the electrical and optical characteristics of their internal layers, etc., are expensive and therefore are not implemented in the biometric sensors that are currently on the market.

Therefore, a life detector is needed that solves the problem derived from the easy simulation and/or fooling of the measured characteristics (the vein pattern in this specific case) at a relatively low cost.

DESCRIPTION OF THE INVENTION

To solve these cost and security problems, an integrated wave guide biometric sensor (optical fibre or flat wave guide) has been designed that uses the evanescent field of the guided light to modify its polarization. The objective of this invention is to detect the presence of a live biological element (hand, finger or any other tissue of a living being that is the biometric recognition element) and differentiate it from one that is not by using the change in polarization of the phase or intensity of the light that is propagated through the guide.

The main basis of this invention is the integrated photonic technology that is used by this device, purpose of this invention and its basic element: the wave guide. A wave guide is an optical structure that confines the light inside its boundaries through total internal reflection (TIR). For this, a high index media is needed (called the guide nucleus) with a refraction index of $n_n$ that is surrounded by other media with a lower refraction index. These can cover the substrate (substrate covering with a refraction index of $n_s$) or the nucleus (nucleus covering with a refraction index of $n_r$). In the majority of cases, the nucleus covering is the external media with a refraction index of $n_e$.

Different types of waveguides exist depending on the number of dimensions in which the light is trapped. In this way, while a flat waveguide traps the electromagnetic radiation in one dimension, the grooved waveguides trap them in two, just like optic fibres. Confinement of the light in three dimensions also exists, where the radiation is trapped and does not propagate (photonic crystals). In this invention, three types of guides can be used as light propagation devices.

When the thickness of the waveguide nucleus is reduced, reaching dimensions close to the confined radiation wavelength, the interference produced by the reflected wave due to the total internal reflection at the nucleus's upper limit and the one reflected in the lower area causes the light to only propagate in certain angles. For each propagation angle allowed, the transversal structure of the electromagnetic field associated to the confined radiation is maintained and the light beam is propagated along the nucleus in what is called the propagation mode. In this way, a propagation mode results from combining the internal total reflection and the constructive interference. The guide that supports only one mode is called single-mode and in the case of an optical fibre, the optical fibre is single-mode.

The simplest optic guide due to its geometry is the conventional flat guide. If the upper medium (nucleus covering) and the lower medium (substrate covering) are the same (same optic constants), the structure is a symmetric flat guide. On the other hand, if the upper and lower media are different, the waveguide will be an asymmetric flat waveguide. In this invention, a waveguide with cylindrical revolution symmetry is used like optical fibre and also flat guides, even photonic crystals as mentioned before. The optical fibre can be symmetrical as well as asymmetrical but in general it will be symmetrical conserving its polarization state.

The physical fundamentals of operation for this device are based on the evanescent field of the guided light inside a waveguide such as a single-mode optical fibre, which must preserve its polarization state. Even though the light inside a waveguide practically propagates confined inside its nucleus (guided modes), there is a part of it (called evanescent field) that extends over to the adjacent media (covering or external medium) and exponentially weakens with the distance to the nucleus (penetration distance). This penetration distance is usually a few hundred nanometres (50-500nm) and enables to detect the changes produced in the external medium when for example, a biological tissue interacts with this previously described evanescent field. The magnitude of the change in light propagation enables to obtain a quantitative measurement of this interaction.

The conventional single-mode optical fibres generally support the fundamental mode propagating through its nucleus. Its polarization state varies as the light propagates inside the fibre. However, if the fibre preserves the polarization and is single-mode, the fundamental modes that propagate through the fibre and as they exit the fibre, will maintain the same polarization state they had entering the fibre.

In this invention, the light attached to the fibre is emitted by a semiconductor laser source connected with fibre. To choose the polarization state (TE or TM) of the light that enters, a polarization controller is used that is also connected to the fibre and controls the polarization. In this way, the polarization state of the light will be controlled as it exits the fibre. If the polarization state is TE (TM) another polarization controller can be subsequently located that polarizes the light that is already TE (TM) polarized in a state with its crossed polarization axis TM (TE) facing the previous one, in such a way that the connected photo detector does not detect any radiation intensity.

It is at this point that the biometric element is introduced, which is going to allow determining if the user is alive or is a replica of the biometric receptor element he possesses. To accomplish this, a section of the covering of the single-mode fibre must be eliminated leaving the external medium to interact with the evanescent field of the guided light. Depending on the type of material it interacts with, the light that propagates through the fibre will change its polarization state so the fibre connected photo-detector will detect the radiation if the light polarization has changed (its state is no longer TE (TM) but a mix of the two). In the case of human epithelial tissue like the one from a finger of the hand, the evanescent field of the guided light interacts with it and its polarization state will change in a determined way depending on its roughness, absorption coefficient and reflection.

Depending on the depth of the evanescent field, the guided light will be able to more or less penetrate inside the live epithelial tissue. The depth of this evanescent field depends on the index contrast between the nucleus and the single-mode waveguide covering through which the light is guided. The greater the contrast and the smaller the diameter of the fibre nucleus, the penetration distance of the evanescent field will be greater. It also depends on the wavelength that is used. In the case of flat single-mode rib type waveguides, a greater penetration depth is obtained for a guide nucleus and covering refraction index of n = 2 and n = 1.46 respectively with silicon substrate. In this case, the light is guided through the nucleus stage of the guide. This stage must be 150nm high and 1-5$\mu$m wide in order to obtain a single-mode behaviour. The thickness of the nucleus in order to reach this maximum sensitivity is 150nm in such a way, that the penetration distance of the evanescent field is approximately 500nm.

In cases where the guided light penetrates a live epithelial tissue at a level deeper than the epidermis layer (d=1.5 mm), it will not only carry live tissue information but will also carry information of the vascular network that is protected by this tissue. This penetration depth is very high (it also depends on the epithelial structure of the user) since the palm of the hand vascular network is located after the dermis, following the epidermis, with lymphatic and blood vessels. In this case, the light guided through the fibre will carry vascular map information, which is another biometric data specific to the person just like the pulse.

This device also attaches electrodes to the biometric element in question. This way, biometric parameters such as bio-impedance, bio-capacitance or bio-reactance are modified depending on the alternate or constant electrical field that is introduced. This modification of the mentioned parameters produces a change in the external media refraction index (in our case of the biometric element in question) and therefore in the effective refraction index of the guide. The polarization is also modified depending on the change in conductivity of the biometric element (human tissue is conductive). This variation is the physical basis in which a first use of this invention is based on since this variation is detected by the evanescent field of the guided light, which varies depending on the electrical field that is applied and the live tissue; these effects being typical of live persons.

A second use of this invention is related with the change in the effective refraction index of the optical fibre guided mode or grooved waveguide as it interacts with the biological recognition element. However, in this case, the light phase that propagates through the fibre/guide is also considered since it mainly uses an optical fibre integrated Mach-Zehnder interferometer, although it can also be used as a flat grooved waveguide with a high evanescent field depth in its detection area.

The laser light attaches to a forked optical fibre that controls the polarization through a laser connected with fibre and a polarization controller that polarizes the light in TE or TM at the forked fibre entrance. This forked fibre can be single-mode or multi-mode but it is desired that it be single-mode in order to achieve a greater sensitivity in the described system. In following and at the output of both forked fibre connectors, two single-mode fibres will be connected that control the polarization, one for each connector output, creating the two optical fibre branches of the biometric interferometer. One of these branches has a detection area that has been obtained by eliminating the fibre covering (cladding) leaving the nucleus uncovered so that the evanescent field of the guided light in this waveguide section is much greater and, therefore, is much more sensitive to the changes in the refraction index of the external medium.

In this interaction area where the biological element (human epithelial tissue of the finger, hand, etc.) is positioned and depending on the depth of the evanescent field, it can more or less penetrate into the tissue interacting with its interior. Due to this interaction, the effective refraction index of the guide mode changes, varying with time (due to the internal biological change of the human organism - pulse, blood pressure, etc, or external like the fingerprint) and is different than the guided mode on the other interferometer branch. Because of this effect, the light that propagates through this interaction area and interacts with the biometric element experiences a phase change with respect to the light from the other branch. Another forked fibre is attached to the interferometer branches by both its ends, in such a way that produces the interference of both light beams that propagate through these branches in the Y joint area of the interferometer. This pattern of interference is collected by a photodiode connected with fibre to the end of the second forked fibre, in such a way that allows detecting the change in phase experienced by the light as it interacts with the biometric element, which is characteristic of the live human being that is being registered or identified.

The device described in this way, can be used as a biometric sensor for personal identification or as a life detection system. Additionally, because it is an integrated photonic device, the waveguide used as propagation media can control the propagated light with conventional photonic controllers like electro-optic or acousto-optic modulators.

Because of this, a third possible use for this invention lies in placing a phase modulator in the above mentioned assembly in such a way that the MZI (Mach-Zehnder Interferometer) is optically modulated displaying a sinusoidal behaviour. This way, if the biometric element is placed on one of the MZI branches, this phase will be modified with respect to the one prior just like the signal amplitude, so this variation will be able to be analysed at the output of the device, which will be characteristic of the biometric element of a specific user.

For the MZI of materials with a high electro-optic index like for example the LiNbO3, the same biometric element can act as an electro-optic modulator, acting as a modulator electrode; so if a difference in potential is introduced between the finger and a metallic electrode, which has the fibre inserted through them, the (transversal) modulation of the optic signal will be obtained at the MZI output, which is characteristic of the biological electrode. Layers of electro-optic, piezo-electric or acousto-optic materials can also be placed over the detection area that in a characteristic way, modify the effective refraction index of the guided mode inside the waveguide when the biometric element is positioned over this area.

BRIEF DESCRIPTION OF THE FIGURES

The following, in a brief way describes a series of drawings that help to better understand this invention. These drawings are expressly related to the embodiment of this invention and provide an non-limiting example of this invention.

- FIG. 1 shows the basics of light propagation inside a waveguide and evanescent field of light guided through its nucleus and through a single-mode optical fibre.
- FIG. 2 shows an experimental assembling of the described system when there is no biometric element present.
- FIG. 3 shows the experimental assembling of the described system when a biometric element is present.
- FIG. 4 shows the electrodes in contact with the biometric element of the user, which supplies an electric field.
- FIG. 5 shows the rib type flat waveguide that can be substituted by the single-mode optical fibre.
- FIG. 6 shows the use of several single-mode optical fibres with different wavelengths and laser sources of λ = 632.8nm corresponding to the near infrared, single-mode optical fibres of λ = 632.8nm corresponding to the near infrared that control the polarization (they include 1300 and 1550nm optical communications) and photo detectors connected with single-mode optical fibre with absorption bands centred on λ = 632.8nm corresponding to the near infrared that control the polarization (they include 1300 and 1550nm optical communications).
- FIG. 7 shows the embodiment of the invented device based on integrated Mach-Zehnder Interferometers (MZI) made of optical fibre or rib type grooved guides.
- FIG. 8 shows the operation of the MZI fibre when an electro-optic modulator is introduced (fig. 8A) and when the biometric element is introduced as the modulator electrode (fig. 8B)

PREFERRED EMBODIMENT OF THE INVENTION

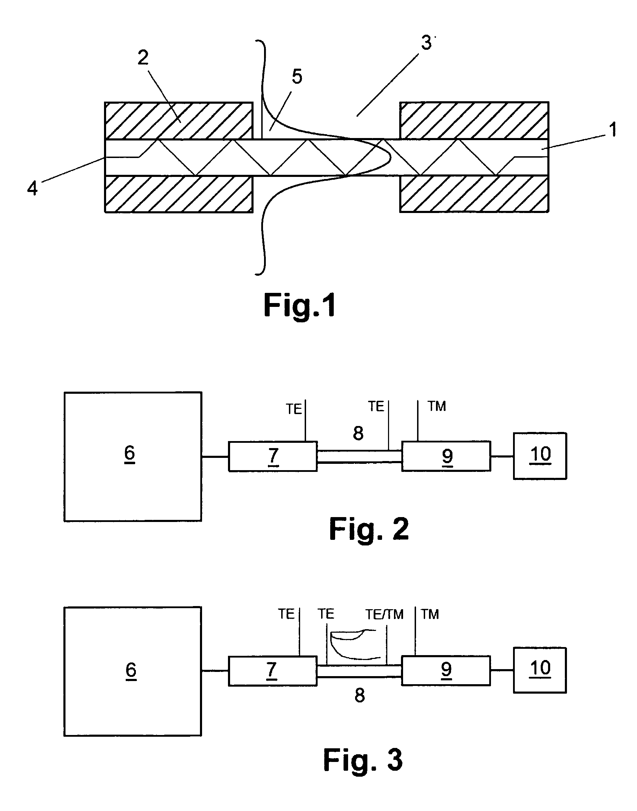

The performance of this invention is based on the evanescent field (5) that propagates through the nucleus (1) of a waveguide that controls the polarization as shown in figure 1. In this figure, the essential mode (4) of a light beam is attached to a single-mode fibre that controls the polarization and propagates through its nucleus. This single-mode optical fibre has a nucleus (1) that is $4\mu m$ in diameter and the diameter of its covering (2) is $150\mu m$. As it propagates through the fibre, the evanescent field (5) of the essential mode interacts with the external media through its detection area (3). The detection area (3) of the fibre is one of its sections without a nucleus covering, which has a longitude of 12mm in this embodiment.

The fundamental element of this invention includes a waveguide (in our case it is an optical fibre or a rib type grooved waveguide) that grants it the peculiarity of an integrated photonic device. The essential mode that propagates through the guide interacts with the external media through its evanescent field (5). Its penetration depth grants sensitivity to the described biometric device. This sensitivity for a rib type flat waveguide (with a guide nucleus stage 4nm high and $4\mu m$ wide and a nucleus thickness of 175nm with a refraction index of $n_n = 2.0$, a substrate covering thickness of $2\mu m$ with a refraction index of $n_s = 1.46$ and an external media refraction index of $n_e = 1.3328$ with a thickness of $10\mu m$) is determined by the following equation [1]:

$$\eta_e \equiv \frac{\partial N}{\partial n_e} \cong \frac{n_e}{N} \frac{k_n^2}{k_n^2 - \gamma_e^2} \frac{\gamma_e}{k_0^2 d_{ef}} \left[ 2 \left( \frac{N}{n_0^2} \right)^2 - 1 \right]^{\rho} \qquad [1]$$

where $k_0 = 2\pi/\lambda$ is the wave vector in a vacuum, $\gamma_e = \sqrt{k_0^2 n_e^2 - \beta^2}$ , $k_n^2 = k_0^2 n_n^2 - \beta^2$ is the wave vector in the guide nucleus and $d_{ef}$ is the effective thickness or the nucleus and is determined by equation [2]:

$$d_{ef} = d_n + \frac{\left[ \left( \frac{N}{n_n^2} \right)^2 + \left( \frac{N}{n_e} \right)^2 - 1 \right]^{-\rho}}{k_o \sqrt{N^2 - n_e^2}} + \frac{\left[ \left( \frac{N}{n_n} \right)^2 + \left( \frac{N}{n_r^2} \right)^2 - 1 \right]^{-\rho}}{k_0 \sqrt{N^2 - n_r^2}} \qquad [2]$$

where $n_e$, $n_n$ and $n_r$ are the refraction indexes of the external media, substrate covering and nucleus respectively, $N$ is the effective refraction index and $d_n$ the real thickness of the nucleus. In equations (1) and (2), the $\rho$ parameter can have a value of $\rho = 0$ if the mode is TE or $\rho = 1$ if the mode is TM.

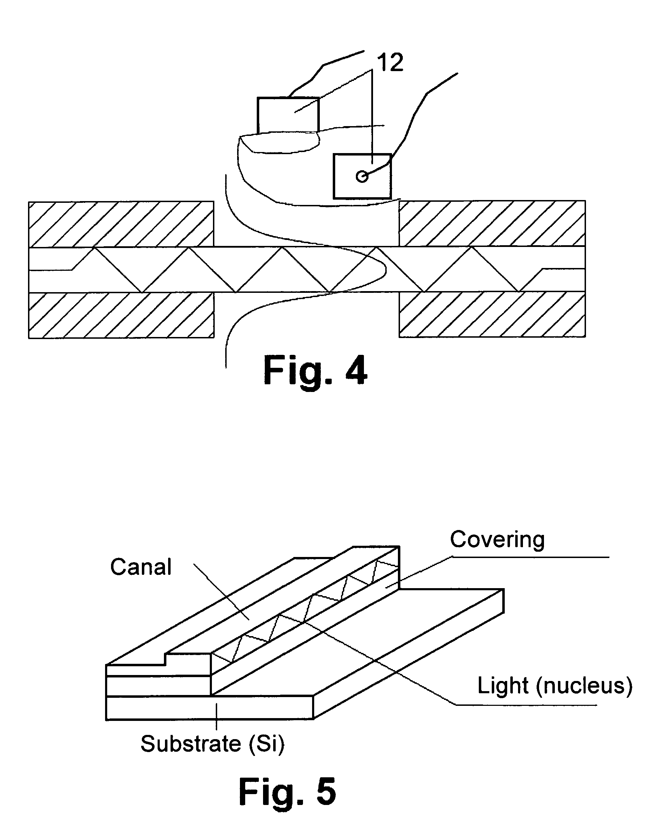

Figures 5a and 5b show the rib type flat waveguide described prior. For this guide (figure 5a), the penetration distance of the evanescent field (figure 5b) is approximately $500\mu m$. In the case of commercial single-mode optical fibres, this penetration distance is somewhat less, around a few hundred nanometres in the best of cases. Therefore, if rib type flat waveguides are used of the described dimensions (figure 5a), the device has a greater sensitivity to optic property changes in the external media like the external refraction index or in the polarization of the beam that interacts in the detecting area (3) of the fibre.

Figures 2 and 3 show a schematic of the invention experimental assembly. A beam of light is attached to a single-mode optical fibre (or grooved waveguide) that controls the polarization (8). This beam of light is polarized with a filbert optic polarization controller (7) in the TM polarization (it can also be in the TE polarization although with this polarization, the essential light mode has less sensitivity). The light beam originates from a source that can be laser or LED (6), centred on a wavelength that is supported by the fibre. In general and for the dimensions of the waveguides described herein, beams with spectral bands centred in the $\lambda=632.8$nm are usually used although wavelengths with emission bands in the near IR can also be supported (in this case, the dimensions of the fibre nucleus diameter is $9\mu$m).

With the light polarization controlled, the beam propagates through the fibre and interacts with the external media when it reaches the detection area (3). In this case, two events can occur:

(i) The external media is the environment (air that surrounds the room). In this case, a polarization change of the beam does not occur as it propagates through the detection area (3) so the light exits the fibre (8) with the same polarization that it had when it entered. A fibre polarization controller (9) is attached to the fibre exit and controls the polarization of the exit beam in such a way, that it is regulated so that it maintains its polarization axis perpendicular (TM polarization) to the first polarizer. Since the beam that exits the fibre has a TE polarization, it will be extinguished by the TM polarization controller so the photo detector that is connected with fibre (10) will not detect any radiation. It may also occur in this event that an artificial material is introduced that does not modify the polarization of the guided light with which it interacts in the detection area (3) of the fibre (the light is only reflected, it is not absorbed by the material). In this case, the photo detector (10) will not detect any radiation either.

(ii) The external medium is the biometric recognition element (11) (finger, palm of the hand, etc.). In this case, (figure 3) a polarization change of the beam occurs as it propagates through the detection area so the polarization of the light that exits the fibre is different (a mix of TE and TM) than what it had when it entered. The same fibre polarization controller of the prior assembly (9) is attached to the fibre exit and controls the polarization of the exit beam, in such a way that it maintains the same polarization axis as in the prior experiment. Since the fibre exit beam is not a pure TE essential mode (it is a mix of TE and TM), it will not be extinguished by the second polarization controller (9) so the photo detector that is connected with fibre (10) will detect the radiation guided through the fibre. It may also occur in this event that an artificial material is introduced (11) (for example a silicon finger for identity theft, etc.) that also modifies the polarization of the guided light that interacts in the detection area of the fibre. In this case, the photo detector (10) will also detect radiation but it is very difficult for the variation in the polarization state of the incident beam to coincide with the one of the biometric element in question.

To avoid any possible fraud in the before mentioned biometric systems, two electrodes (12) supply an electric field to the biometric recognition element. In this case, the finger that appears in figure 4 is subjected to an electrical difference in potential between the two contact points and the two electrodes (12) in such a way, that increasing the applied voltage will increase the variation of the optic parameters (refraction index, absorption, reflection, transmittance, scattering and polarization) as the guided light interacts with human tissue. For high electrical fields, the change in the biometric element electrical properties is greater and penetrates deeper inside it and is detected by the evanescent field of the guided light. In the case of the invention described herein, the studied optical parameter is the polarization, phase and amplitude of the guided light inside the guide and its variation is characteristic of the human tissue and the liquid media inside it and therefore of the specific user.

As described, this invention can not only be used as a means to detect life but as a biometric identification system as well. It can be used in conjunction with biometric identification systems (of vein patterns, fingerprint, geometrical recognition of the hand, etc.) to detect that the user is alive and has not been substituted by artificial material or it can be used as a biometric system to complement another that also obtains biometric information since it is an integrated photonic device of small size enabling it to be coupled to a vein scanner or a fingerprint recognition system among others.

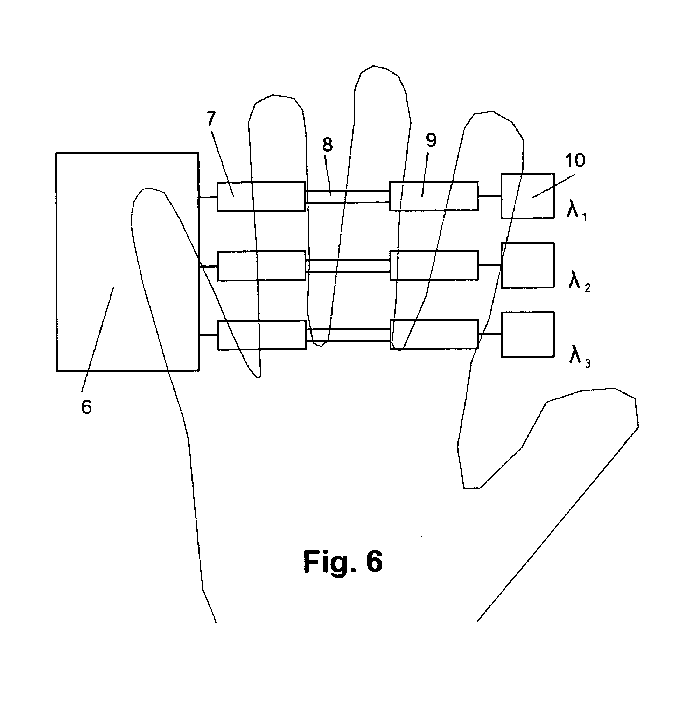

A source with an emission band centred on a characteristic wavelength depending on the wavelength supported by the fibre has been used in the described invention. As mentioned before, the wavelengths commonly used are $\lambda=632.8$nm and the ones corresponding to the near infrared ($\lambda=700$nm-1600nm) due to their high ability to penetrate human tissue. Because of this, several optical fibres instead of just one can be used in such a way, that there are several wavelengths that interact with the biological element. Figure 6 describes this new experimental assembly where for each single-mode optical fibre, there is one light source, two polarization controllers and two photo detectors. The detection areas (3) must all be placed at the same distance from the respective sources. This addition to the invention grants greater security and feasibility to the identification system. As can be seen, the same biometric recognition element interacts with each of the detection areas of each fibre. The wavelengths used are within the near infrared band and are commonly used in optical communications.

In the case that grooved rib type flat waveguides are used, its sensitivity varies depending on the thickness of the guide nucleus just like its polarization control. In fact, for guide nucleus thicknesses of 75nm, only TE modes with high sensitivity propagate through them. This way, the experimental system is simplified and the first polarization controller is not needed. The second controller must have its polarization axis perpendicular to the polarization axis of the light that propagates (TM polarization) so that the photo detector does not detect any radiation (when a biometric element is not present).

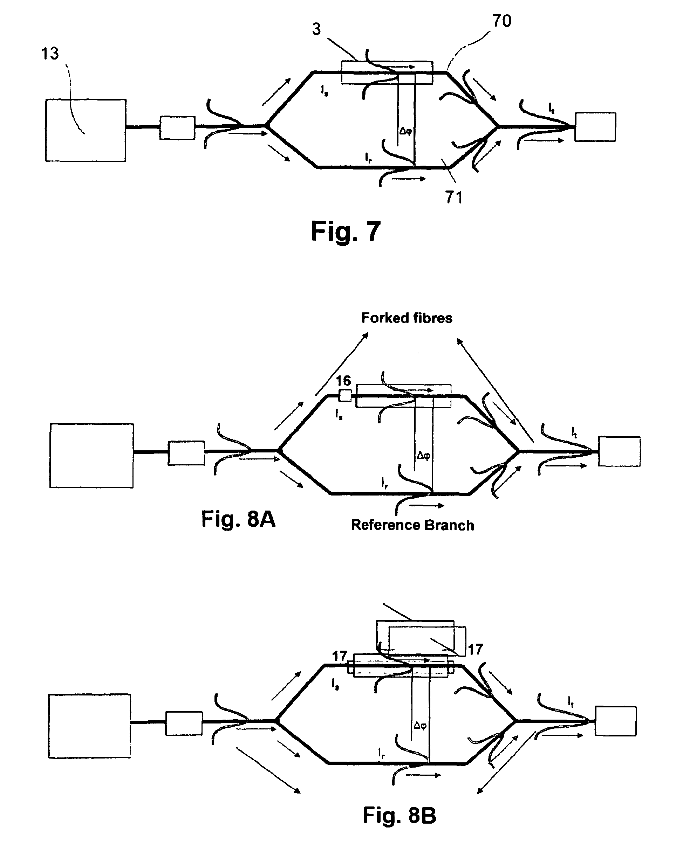

The basis for interaction between the electromagnetic radiation and the biometric element can be also used to study the change in phase of the guided light. Several methods can be used to study this optical property, one of them being the integrated Mach-Zehnder interferometer. The integrated Mach-Zehnder interferometer is an optical device whose configuration is shown in figure 7. The light propagates through a waveguide, in our case through an optic fibre or grooved rib type waveguide that divides itself into two beams by using a Y divider. Each beam travels through a branch of the interferometer (detection and reference branch) to later re-unite inside an exit guide by using another Y connection, producing the interference in both beams. In the detection branch, the evanescent field interacts with the biological interaction to be detected, which induces a change in phase of the light that travels through this branch with respect to the one that travels through the reference branch. At the sensor exit, both beams will interfere, which produces a variation in the intensity which is a function of cos($\Delta\varphi$), $\Delta\varphi$ representing the phase difference produced in the detection branch. Therefore, the device output signal will be calculated by equations [3] and [4]:

$$ I_T = I_S + I_R + 2\sqrt{I_S I_R} \cos \Delta\varphi_S \qquad [3] $$

$$ \Delta\varphi_S = \frac{2\pi}{\lambda} \cdot l \cdot \Delta N_{eff} \qquad [4] $$

where $I_T$ is the intensity of the light exiting the MZI, $I_S$ and $I_R$ respectively are the intensities of the lights at the detection and reference branches and $\Delta\varphi_s$ the phase variation between the light travelling through the detection and the reference branches. This phase variation depends on the longitude of the detection area, the working wave $\lambda$ and the difference between the effective refraction indexes of both branches. In our case, the detection area (3) is the nucleus of the single-mode optic fibre that controls the polarization (or of a grooved waveguide) that is exposed to the exterior media. It is in this area of the MZI where the external refraction index changes are detected, which is the new layer of fibre covering in this area so that the integrated biosensor element receptor that interacts with the evanescent field of the guided light (the refraction index of human tissue is less than the one from the single-mode optical fibre) acts as the new guide covering and interacts with the evanescent field of the guided light. Depending on the penetration depth of this evanescent field and of the polarization used (TE or TM), the vital biological dynamic changes are detected (blood pulse, blood pressure, etc) and the tissue and cell characteristics with greater or less sensitivity by detecting the phase variation of the guided light. Additionally, the new biological covering depends on the human fingerprint or on the roughness of the tissue, which is characteristic of the individual that is being identified. An electric field can also be applied to the biometric element in order to modify the phase of the guided light.

As discussed, the MZI can be made of single-mode optical fibres, which control the polarization (especially inside the interaction area) or by grooved waveguides. The later, for the described TIR configurations (figure 5), possess greater sensitivity than commercial optical fibres (plastic, $SiO_2$, doped, etc.). However, they have the disadvantage of having to be very clean because the great depth of the evanescent field causes the guided mode to be absorbed by the covering or exterior media if it does not have a low degree of roughness (dust particles, grease, etc.).

Currently, the great advances produced in photonics allow for control of light without electrical manipulation. This way, changes in amplitude or phase of the guided light can be induced and can be detected by optical devices like the MZI. As shown in figure 8, the MZI detection branch is connected to a $LiNbO_3$ phase electro-optical modulator (16) (although it can also be thermal-optical or acousto-optical) to modulate the light and in this way obtaining a modulated optical signal at the MZI output. If the biometric element is placed over its detection area, this optical modulation changes and this change, which is characteristic of the human being can be detected.

In the same way, the optical modulation can be performed if the biometric element is placed over the detection area (nucleus) of an MZI with fibres or waveguides manufactured with materials which possess a high electro-optical coefficient (for example, LiNbO3, reference 17). In this case, the biometric element acts as a biological electrode for the modulator and the optical signal at the output will depend on the characteristic biological element. The voltage of the electrode that is placed across from the fibre is around tens of volts.

**Claims**

1. Life detector device of the type used for personal identification that uses the evanescent field of the guided light (5) inside a waveguide that preserves the polarization, **characterised in that** this waveguide (8) is configured so that the interaction of the light with the biometric element provokes a change in the polarization of the light as it exits the fibre with respect to the polarization it has when it enters, which is detected by a photo-detector connected to the fibre (10,15), and this change in polarization of the guided light is characteristic of the user that is being identified.

2. Device in accordance with claim 1 where the waveguide (8) is a single mode optical fibre whose nucleus is between 4 and 9$\mu$m in diameter and whose covering is 150$\mu$m in diameter.

3. Device in accordance with claims 1 and 2, where this device at the same time includes:

   (i) initial polarized controller media connected with single-mode optical fibre (7,9) with their polarization axis crossed;
   (ii) a light source (6) or light sources that emit electromagnetic radiation with a wavelength of 632.8nm or wavelengths in the near infrared;
   (iii) at least one single-mode optical fibre that supports wavelengths of $\lambda1$=632.8 nm or centred in the near IR (14); and
   (iv) at least one photo detector connected with single-mode optical fibre (10,15) with absorption bands centred on $\lambda$ = 632.8nm and wavelengths in the near IR.

4. Device in accordance with claims 1 and 2 that additionally includes several metallic electrodes (12) that subject the biological element to be recognized to an electric field in such a way that the polarization of the guided light that interacts with this biological element receptor is modified in a characteristic and unique way.

5. Device in accordance with claim 3 where the photo detectors connected with single-mode optical fibre incorporate at least one integrated mach-Zehnder interferometer of single-mode optical fibre (15) that controls the polarization with an open detection area (3), which is the nucleus of the fibre and is where the biometric element is placed, which is configured to measure the optical intensity variation produced by the change in phase of the guided light that interacts with the biometric element.

6. Detector in accordance with claim 5 where the detection of the biological parameters is performed by measuring the phase change experimented by the guided light that interacts with the biometric element placed in the detection area (3) of the Mach-Zehnder interferometer.

7. Device in accordance with claims 5 and 6 where an electric field is induced into the biometric element to be recognized, by means of metal electrodes (12), in such a way that the change in phase detected depends on the characteristics of the biometric element.

8. Detector in accordance with claims 5 to 7 that has greater or less sensitivity depending on whether the light is respectively TM or TE polarized.

9. Detector in accordance with claims 5 to 8 that incorporates an electro-optical modulator that modulates the guided optical signal in such a way that the modulated optical signal is modified by the biometric element in a characteristic way and is detected by the connected photodiode (15).

10. Detector in accordance with claims 5 to 9 where the characteristic biometric element is placed over the MZI sensor area and acts like an electrode that has been subjected to a difference in potential with respect to another electrode across from it and between which the optical fibre is positioned (transversal phase modulation).

11. Detector in accordance with claims 5 to 10 where the electrodes (12) are configured by the characteristic biometric element and another metallic one located across from the biometric element in such a way that the guided optical signal in the MZI is modulated by the biological optical modulator in a unique way according to the unique characteristics of the biometric element.

12. Detector in accordance with claim 1 where the waveguide is an integrated rib type grooved waveguide with a transmission wavelength of $\lambda$=632.8nm and other wavelengths in the near IR, which additionally incorporates:

a refraction index nucleus of n=2 and thicknesses of 75nm, 150nm and 200nm;

a stage in the nucleus of the guide that is between 3 and 6$\mu$m wide and 1 to 3nm high that give it its single-mode behaviour;

a substrate covering of 2$\mu$m thick and a refraction index of n=1.46;

a nucleus covering of 2$\mu$m thick and a refraction index of n=1.46; and

a detection area (3) 15mm long, through which the evanescent field of the guided light interacts with the biometric element.

13. Detector in accordance with claim 13 where the integrated rib type grooved waveguide has a thickness of 200nm to reach a high sensitivity in TM, 150nm to reach maximum sensitivity in TM, 75nm to reach maximum sensitivity in TE and null sensitivity in TM, since the TM modes are not supported by the guide.

14. Detector in accordance with claims 12 and 13 that incorporates Mach-Zehnder interferometers and straight guides with sensor areas covered with electro-optical, piezo-electric or acousto-optic materials over which the biometric element is placed and the effective refraction index of the TE or TM guided modes is modified with the corresponding change in intensity detected by the connected photodiode.

15. Integrated photonic life detection and personal identification system that incorporates the detector of claims 1 through 14 and uses single-mode optical fibres and grooved waveguides attached to light sources with different emission wavelengths centred on 632.8nm or in the near IR.

**Fig.1**

**Fig. 2**

**Fig. 3**

**Fig. 4**

**Fig. 5**

**Fig. 6**

**Fig. 7**

**Fig. 8A**

**Fig. 8B**

# INFORME DE BÚSQUEDA INTERNACIONAL

| Solicitud internacional Nº |
| --- |
| PCT/ ES 2009/070361 |

**A. CLASIFICACIÓN DEL OBJETO DE LA SOLICITUD**

Ver hoja adicional

De acuerdo con la Clasificación Internacional de Patentes (CIP) o según la clasificación nacional y CIP.

**B. SECTORES COMPRENDIDOS POR LA BÚSQUEDA**

Documentación mínima buscada (sistema de clasificación seguido de los símbolos de clasificación)

G01N, A61B, G01J

Otra documentación consultada, además de la documentación mínima, en la medida en que tales documentos formen parte de los sectores comprendidos por la búsqueda

Bases de datos electrónicas consultadas durante la búsqueda internacional (nombre de la base de datos y, si es posible, términos de búsqueda utilizados)

INVENES, EPODOC, WPI, INSPEC

**C. DOCUMENTOS CONSIDERADOS RELEVANTES**

| Categoría* | Documentos citados, con indicación, si procede, de las partes relevantes | Relevante para las reivindicaciones Nº |
| --- | --- | --- |
| A | JP 2007313286 A (MATSUSHITA ELECTRIC WORKS, LTD. et al.) 06.12.2007, todo el documento. | 1-3, 5, 6, 8, 12-15 |
| A | US 6301375 B1 (CHOI, H.) 09.10.2001, todo el documento. | 1-3, 5, 6, 8, 12-15 |
| A | US 2008/0211628 A1 (HASHIMOTO, G. et al.) 04.09.2008, todo el documento. | 1-3, 5, 6, 8, 12-15 |
| A | JP 2006068328 A (SONY CORP.) 16.03.2006, todo el documento. | - |
| A | JP 2006288872 A (CANON KK) 26.10.2006, todo el documento. | - |

☐ En la continuación del Recuadro C se relacionan otros documentos    ☒ Los documentos de familias de patentes se indican en el Anexo

| * | Categorías especiales de documentos citados: | "T" | documento ulterior publicado con posterioridad a la fecha de presentación internacional o de prioridad que no pertenece al estado de la técnica pertinente pero que se cita por permitir la comprensión del principio o teoría que constituye la base de la invención. |
| --- | --- | --- | --- |
| "A" | documento que define el estado general de la técnica no considerado como particularmente relevante. | | |
| "E" | solicitud de patente o patente anterior pero publicada en la fecha de presentación internacional o en fecha posterior. | | |
| "L" | documento que puede plantear dudas sobre una reivindicación de prioridad o que se cita para determinar la fecha de publicación de otra cita o por una razón especial (como la indicada). | "X" | documento particularmente relevante; la invención reivindicada no puede considerarse nueva o que implique una actividad inventiva por referencia al documento aisladamente considerado. |
| "O" | documento que se refiere a una divulgación oral, a una utilización, a una exposición o a cualquier otro medio. | "Y" | documento particularmente relevante; la invención reivindicada no puede considerarse que implique una actividad inventiva cuando el documento se asocia a otro u otros documentos de la misma naturaleza, cuya combinación resulta evidente para un experto en la materia. |
| "P" | documento publicado antes de la fecha de presentación internacional pero con posterioridad a la fecha de prioridad reivindicada. | | |
| | | "&" | documento que forma parte de la misma familia de patentes. |

| Fecha en que se ha concluido efectivamente la búsqueda internacional. | Fecha de expedición del informe de búsqueda internacional |
| --- | --- |
| 10.Diciembre.2009     (10.12.2009) | **14 de Diciembre de 2009 (14/12/2009)** |
| Nombre y dirección postal de la Administración encargada de la búsqueda internacional     O.E.P.M. <br><br> Paseo de la Castellana, 75 28071 Madrid, España. <br> Nº de fax 34 91 3495304 | Funcionario autorizado <br> O. González Peñalba <br><br><br> Nº de teléfono +34 91 349 54 75 |

Formulario PCT/ISA/210 (segunda hoja) (Julio 2009)

INFORME DE BÚSQUEDA INTERNACIONAL

Información relativa a miembros de familias de patentes

| Solicitud internacional Nº |
|---|
| PCT/ES 2009/070361 |

| Documento de patente citado en el informe de búsqueda | Fecha de Publicación | Miembro(s) de la familia de patentes | Fecha de Publicación |
|---|---|---|---|
| JP 2007313286 A | 06.12.2007 | NINGUNO | ------------ |
| US 6301375 B | 09.10.2001 | JP 10295674 A<br>JP 4132211 B<br>KR 100259475 B | 10.11.1998<br>13.08.2008<br>15.06.2000<br>15.06.2000<br>15.06.2000 |
| US 2008211628 A | 04.09.2008 | US 7550707 B<br>JP 2008217190 A<br>JP 4306744 B | 23.06.2009<br>18.09.2008<br>05.08.2009 |
| JP 2006068328 A | 16.03.2006 | NINGUNO | ------------ |
| JP 2006288872 A | 26.10.2006 | NINGUNO | ------------ |

Formulario PCT/ISA/210 (anexo_familia de patentes) (Julio 2009)

**INFORME DE BÚSQUEDA INTERNACIONAL**

Solicitud internacional Nº

PCT/ ES 2009/070361

CLASIFICACIÓN DEL OBJETO DE LA SOLICITUD

*G01N 21/21* (2006.01)
*G01N 21/45* (2006.01)
*A61B 5/117* (2006.01)

Formulario PCT/ISA/210 (hoja adicional) (Julio 2009)

**EP 2 413 129 A1**

## REFERENCES CITED IN THE DESCRIPTION

**Patent documents cited in the description**

- US 6813010 B